(19) Europäisches Patentamt
European Patent Office
Office européen des brevets

(11) **EP 1 651 757 B1**

(12) **EUROPÄISCHE PATENTSCHRIFT**

(45) Veröffentlichungstag und Bekanntmachung des Hinweises auf die Patenterteilung:
**21.11.2007 Patentblatt 2007/47**

(21) Anmeldenummer: **04763894.5**

(22) Anmeldetag: **06.08.2004**

(51) Int Cl.:
***C12N 5/08*** *(2006.01)*  ***A61K 35/28*** *(2006.01)*

(86) Internationale Anmeldenummer:
**PCT/EP2004/008865**

(87) Internationale Veröffentlichungsnummer:
**WO 2005/015151 (17.02.2005 Gazette 2005/07)**

(54) **AUFREINIGUNGSVERFAHREN FÜR MESENCHYMALE STAMMZELLEN**

METHOD FOR PURIFYING MESENCHYMAL STEM CELLS

PROCEDE DE PURIFICATION DE CELLULES SOUCHES MESENCHYMATEUSES

(84) Benannte Vertragsstaaten:
**AT BE BG CH CY CZ DE DK EE ES FI FR GB GR HU IE IT LI LU MC NL PL PT RO SE SI SK TR**

(30) Priorität: **06.08.2003 DE 10336152**

(43) Veröffentlichungstag der Anmeldung:
**03.05.2006 Patentblatt 2006/18**

(73) Patentinhaber: **Nephrogen LLC**
**Salt Lake City, UT 84108 (US)**

(72) Erfinder:
• **LANGE, Claudia**
  **22529 Hamburg (DE)**
• **ZANDER, Axel, Rolf**
  **22359 Hamburg (DE)**

(74) Vertreter: **Weber-Quitzau, Martin**
**Uexküll & Stolberg**
**Patentanwälte**
**Beselerstrasse 4**
**22607 Hamburg (DE)**

(56) Entgegenhaltungen:
**WO-A-01/94541**          **WO-A-97/40137**
**US-A- 5 197 985**        **US-A- 5 965 436**

• **"Percoll: Methodology and Applications" 2001, AMERSHAM BIOSCIENCES , XP002316866 Gefunden im Internet: URL:http: //www.cellseparation.nu/media/18-1115-69AC.pdf> Abbildung 11 das ganze Dokument**
• **PHINNEY D G ET AL: "Donor variation in the growth properties and osteogenic potential of human marrow stromal cells" JOURNAL OF CELLULAR BIOCHEMISTRY, Bd. 75, Nr. 3, 1. Dezember 1999 (1999-12-01), Seiten 424-436, XP002316863 ISSN: 0730-2312 in der Anmeldung erwähnt**
• **PITTENGER M F ET AL: "Multilineage potential of adult human mesenchymal stem cells" SCIENCE, Bd. 284, Nr. 5411, 2. April 1999 (1999-04-02), Seiten 143-147, XP002316864 ISSN: 0036-8075 in der Anmeldung erwähnt**
• **BRUDER S P ET AL: "Growth kinetics, self-renewal, and the osteogenic potential of purified human mesenchymal stem cells during extensive subcultivation and following cryopreservation" JOURNAL OF CELLULAR BIOCHEMISTRY, Bd. 64, Nr. 2, 1997, Seiten 278-294, XP002316865 ISSN: 0730-2312 in der Anmeldung erwähnt**

**Beschreibung**

Die vorliegende Erfindung betrifft ein Verfahren zur Aufreinigung mesenchymaler Stammzellen (MSC; CD34-negative, plastikadhärent wachsende, fibroblastoide Zellen), das dem Stand der Technik entsprechende Zellausbeuten liefert, wobei sich die erhaltenen Zellen durch eine gesteigerte Proliferationskapazität unter gleichzeitiger Beibehaltung der Multipotenz und der typischen Antigen-Charakteristika auszeichnen. Damit stehen erstmals mesenchymale Stammzellen zur Verfügung, die sich gegenüber dem Stand der Technik deutlich besser vermehren lassen und darüber hinaus auch nach längerer Kultivierung noch in drei mesenchymale Richtungen differenzieren lassen.

Stand der Technik

Mesenchymale Stammzellen (MSC) werden aus dem adulten Knochenmark gewonnen. Für die Generierung der MSC gibt es weltweit die verschiedensten Protokolle, die sich in Parametern wie z.B. Anreicherung, verwendetes Medium und Auswahl des fötalen Kälberserums unterscheiden.

Ein wichtiger Schritt zur Anreicherung der MSC besteht in der Abreicherung der Zellen aus dem Knochenmark, die ein Proliferations- und Differenzierungspotential, wie es für MSC beschrieben wurde, nicht mehr besitzen (z.B. Erythrozyten, Granulozyten). Eine weit verbreitete Methode in der Hämatologie ist die Dichtegradientenzentrifugation mit den isotonischen Lösungen Ficoll® oder Percoll®. Diese Separationsmethode begründet sich darauf, daß jede definierte Zelle eine bestimmte Dichte besitzt und sich während der Zentrifugation in Richtung der Dichte des Trennmediums bewegt, an der ihr isopycnischer Punkt liegt.

Bei einer Dichte von 1,077 g/ml werden routinemäßig mononukleäre Zellen (MNC) an der Grenze zwischen Probe und Dichtelösung angesammelt, wohingegen die Erythrozyten und Granulozyten am Boden des Röhrchens konzentriert werden. Diese. Dichte wird von vielen Autoren auch für die Anreicherung von MSC aus dem Knochenmark genutzt (Azizi et al., Proc Natl Acad Sci USA 95 (1998):3908-13; Phinney et al., J Cell Biochem 75 (1999): 424-36; DiGirolamo et al., Br J Haematol 107 (1999):275-81; Muraglia et al., J Cell Science 113 (2000):1161-66; Colter et al., Proc Natl Acad Sci USA 97 (2000):3213-18, Proc Natl Acad Sci USA 98 (2001):7841-45 ; Quirici et al., Exp Hematol 30 (2002): 783-91). Keiner der Autoren hat vergleichende Studien mit Zellen durchgeführt, die über andere Dichten angereichert wurden, und es bleibt unklar, inwieweit in der Population der MNC Zellen mit stimulierenden oder supprimierenden Eigenschaften enthalten sind. Morphologisch werden von Azizi et al., loc. cit., zwei Typen von Zellen gezeigt: abgeflachte und langgestreckte Zellen. Von Muraglia et al., loc. cit., wurden drei Phänotypen von Klonen gezeigt: fibroblastenartig verlängerte Zellen, große abgeflachte Zellen und schmale sternförmige Zellen. Es ist bekannt, dass Kulturen, die eine hohe Anzahl abgeflachter Zellen enthalten, langsamer proliferieren bzw. sukzessive das Wachstum einstellen. Späteres Auswachsen flacher Zellen kann nicht mehr beeinflußt werden.

Die Anzahl der Verdopplungen schwankt bei den Autoren zwischen 4 und nahezu 50 (Colter et al., Proc Natl Acad Sci USA 97 (2000):3213-18; die Zahl 50 ist jedoch fraglich, da aus 20 ml Knochenmark kummulativ $10^{13}$ Zellen generiert wurden und selbst bei nur einer Zelle als Ausgangszahl 43 Verdopplungen erreicht worden wären) und hängt von der Qualität des SpenderKnochenmarks ab. Außerdem sind die im Stand der Technik vorhandenen Angaben von Passagen vorsichtig zu interpretieren. Meist werden 5 x $10^3$ Zellen/cm$^2$ ausplattiert und die Zellen bei nahezu Konfluenz geerntet und gezählt. Pro Passage ergeben sich ca. 2 Verdopplungen.

Zellen, die mit d = 1,077 g/ml aufgereinigt werden, differenzierten in vitro in die osteo-, chondro- und adipogene Richtung zumindest in frühen Passagen. In wenigen Studien (DiGirolamo et al., loc. cit.) wurde die Differenzierungsfähigkeit der MSC bis zur Beendigung der Kulturen untersucht. Muraglia et al., loc. cit., zeigten anhand von klonierten MSC, dass die osteogene Differenzierung auch in späten Passagen nicht verlorenging, die adipogene und auch die chondrogene Differenzierung hingegen nicht in allen Klonen bis zum Ende der Kulturen erhalten blieb. Die adipogene Differenzierung wird zuerst von den Zellen eingestellt, ebenso können die Zellen nicht bis in die letzten Passagen in Chondrozyten differenziert werden. Das osteogene Differenzierungsvermögen scheint eine generelle Eigenschaft von MSC zu sein und geht meist bis zum Stillstand des Zellwachstums nicht verloren.

Von anderen Autoren wird eine Dichte von 1,073 g/ml zur Anreicherung verwendet (Majumdar et al., J Cell Physiol 176 (1998):57-66; Mackay et al., Tissue Engineering 4 (1998):415-28; Pittenger et al., Science 284 (1999):143-7; Mosca et al., Clin Orthop Rel Res 379S (2000):S71-90; Koc et al., Bone Marrow Transpl 30 (2002):215-22; Toma et al., Circulation 105 (2002):93-8) und die Zellen als "low density" bezeichnet. Pittenger et al., loc. cit., beschreiben die isolierten Zellen als morphologisch uniform, in den in der Publikation vorhandenen Abbildungen sind jedoch auch abgeflachte Zellen zu sehen, deren Funktion nicht weiter kommentiert wird.

Die über eine Dichte von 1,073 g/ml angereicherten Zellen waren in drei Differenzierungsassays (adipo-, osteo- und chondrogene Differenzierung) positiv ohne Spontandifferenzierung. Die Proliferationsfähigkeit ist auch bei diesen Zellen abhängig von sorgfältig ausgewählten Seren. Nach 2 Passagen werden 5-37,5 x $10^7$ Zellen generiert, was Daten mit konventionell separierten Zellen entspricht, also keinen Vorteil für diese Methode implementiert.

Eine dritte Gruppe von Autoren benutzt die sehr aufwendige Methode eines vorgeformten kontinuierlichen Gradienten aus 70% Percoll® für die Separation des Knochenmarks (Lennon et al., In Vitro Cell Dev Biol 32 (1996):602-11; Bruder et al., J Cell Biochem 64 (1997):278-94; Jaiswal et al., J Cell Biochem 64 (1997): 295-312; Fleming et al., Developm Dynamics 212 (1998): 119-32; Liechty et al., Nat Med 6 (2000):1282-6). Nach Zentrifugation werden die ersten 25% als "low density"-Zellen für die Generierung von MSC benutzt und die gepoolte Dichte als 1,030 g/ml angegeben, wobei bei diesem Wert ein Nacharbeiten bei der vorhandenen Datenlage nicht nachvollziehbar ist.

Der Nachteil der letzgenannten Methode besteht ferner darin, dass die Vorbereitung von kontinuierlichen Gradienten zeit- und materialaufwendig ist und von vielen Labors nicht durchgeführt werden kann (es wird eine Zentrifuge mit 20.000 g benötigt). Die mit dieser Methode aufgereinigten Zellen erhalten ebenso wie die oben beschriebenen ihr osteogenes Differenzierungspotential während aller Subkultivierungen und sind positiv für MSC-spezifische Oberflächenantigene (Bruder et al., loc. cit.). Mit zunehmender Kultivierungsdauer wird auch bei diesen Zellen über eine Zunahme des flachen, ausgebreiteten Phänotyps berichtet, die Zellen akkumulieren Debris und Streßfibers (Actin), bis die Kultur letztendlich komplett degeneriert (Bruder et al., loc. cit.).

Aus den im Stand der Technik bekannten Ergebnissen wird klar, dass das Auswachsen wenig mitotischer Zellen offensichtlich mit keiner Separationsmethode zu verhindern ist. Vielmehr spielen hier die Kultivierungsbedingungen eine große Rolle. Hier ist die sorgfältige Selektion der Population mit den ausgeprägtesten Proliferationseigenschaften gefragt.

**Aufgabe** der vorliegenden Erfindung ist es daher, ein neues Verfahren zur Aufreinigung mesenchymaler Stammzellen bereitzustellen, dass die aus dem Stand der Technik bekannten Nachteile nicht aufweist. Insbesondere soll das Ziel der Aufreinigung sein, mit Beginn der Kultur die Anzahl abgeflachter Zellen zu minimieren, da von diesen Zellen bekannt ist, dass sie langsamer proliferieren bzw. sukzessive das Wachstum einstellen.

Zur **Lösung** wird ein Verfahren vorgeschlagen, bei dem man mesenchymale Stammzellen aus Knochenmark durch Dichtegradientenzentrifugation isoliert, wobei man die Zellen aus einer Fraktion mit einer gegenüber dem Stand der Technik geringeren Dichte von < 1,073 g/ml, vorzugsweise ≤ 1,070 g/ml, isoliert.

Bevorzugt sind 1,050 g/ml bis 1,070 g/ml, und besonders bevorzugt ist eine Dichte von 1,068 g/ml.

Überraschenderweise wurde festgestellt, dass sich bei Verwendung der erfindungsgemäßen Dichte von < 1,073 g/ml, insbesondere bei 1,068 g/ml, Zellen anreichern lassen, die - gegenüber Zellen, die bei höheren Dichten isoliert wurden - eine gesteigerte Proliferationskapazität unter Beibehaltung der Multipotenz und der typischen Antigen-Charakteristika besitzten. Morphologisch zeigen die erfindungsgemäßen mesenchymalen Stammzellen für einen verlängerten Zeitraum eine fibroblastoide Form, bevor die Kultur zum Stillstand kommt.

Die Erfindung betrifft gemäß einer besonders bevorzugten Ausführungsform die Verwendung einer Lösung von Ficoll® oder Percoll® der Dichte 1,068 g/ml zur Durchführung einer Dichtegradientenzentrifugation zur Isolierung mesenchymaler Stammzellen aus Knochenmark.

Gegenstand der Erfindung sind ferner die nach dem beschriebenen Verfahren erhaltenen mesenchymalen Stammzellen (bzw. eine Präparation, die ausschließlich oder überwiegend - d.h. zu mindestens 70%, 80% oder vorzugsweise zu mindestens 90% - diese Zellen enthält) sowie diese Zellen enthaltende pharmazeutische Präparate.

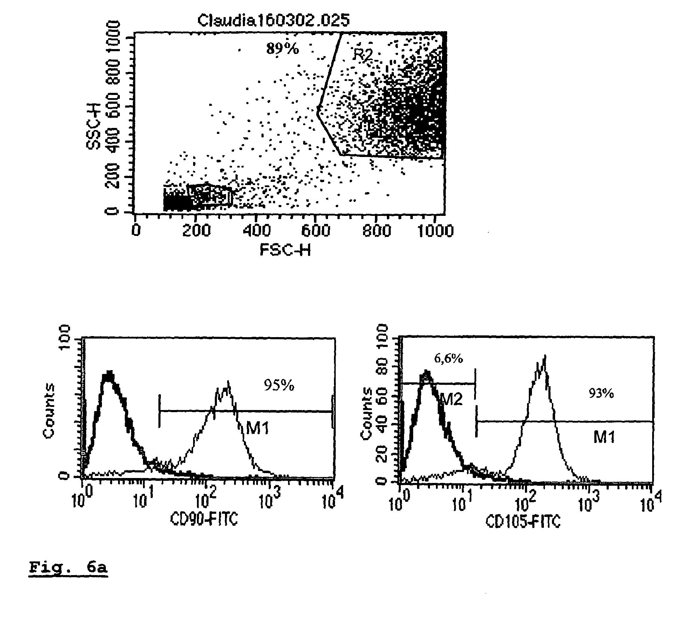

Die erfindungsgemäßen Zellen exprimieren die typischen Oberflächenmarker mesenchymaler Stammzellen (CD90, CD105, CD59). Bei Untersuchung der Expression über viele Passagen bis zum Ende der Kulturen und mit zunehmender Kultivierungsdauer wird eine Abnahme (von > 90% bis ca. 60% im Durchschnitt bei höheren Passagen) der Expression positiver Marker wie CD90 und CD105 gefunden und keine Zunahme der Expression hämatopoetischer Marker wie CD45 und CD34. Die Abnahme der Expression mesenchymaler Marker korreliert mit der beschriebenen Alterung der Zellen (vgl. Fig. 6a versus 6b).

Im Rahmen der Erfindung wurden die nach dem beschriebenen Verfahren erhaltenen Zellen dünn ausgesät (ca. 500 Zellen/cm$^2$) und verdoppelten sich bis zur nächsten Passage ca. 3,3 mal mehr als Zellen, die nach herkömmlichen Verfahren isoliert und entsprechend hoch ausgesät (ca. 5.000 Zellen/cm$^2$) worden waren (für die ein Wert von 2 angegeben ist). Insgesamt wurden erfindungsgemäß bis zu 45 Verdopplungen erreicht.

Alle untersuchten Fraktionen konnten bis zum Ende der Kultur in die drei untersuchten mesenchymalen Richtungen differenziert werden:

- Die Differenzierung in Osteoblasten (**osteogene** Differenzierung; Induktion nach Jaiswal et al., loc. cit.) blieb unbeeinflußt durch die zunehmende Passagenzahl. Gegen Ende der Kulturen differenzierten die Zellen zeitweise spontan in Calcium-sektretierende Zellen, was diese Erkenntnis unterstreicht.
- Die **adipogene** Differenzierung (Induktion nach Pittenger et al., loc. cit.) hingegen nahm mit zunehmender Passagenzahl ab und war letztendlich nur noch in einzelnen Zellen nachweisbar (von ca. 50% nach zwei Wochen Differenzierungsinduktion bis etwa 1-2% bei höheren Passagen).
- Die Differenzierung in die **chondrogene** Richtung (Induktion modifiziert nach Shakibaei et al., Cell Biol Internat 21 (1997):75-86) wurde ebenfalls mit zunehmender Passagenzahl geringer, jedoch nicht im gleichen Ausmaß wie die adipogene Differenzierung (von ca. 90% nach einer Woche Differenzierungsinduktion bis ca. 15% bei höheren Passagen), und ein Teil (ca. 10-20%) der Zellen aller Fraktionen zeigte die typische Proteoglykan-Anfärbung. In Zellen höherer Dichten (d.h. von $\geq$ 1,077) war diese chondrogene Differenzierungsfähigkeit weniger ausgeprägt als in denen geringer Dichten (d.h. < 1,077 g/ml).

Bei dem erfindungsgemäßen Verfahren zur Isolierung mesenchymaler Stammzellen können beliebige (isotonische) Gradienten eingesetzt werden, wie z.B. Ficoll®-Gradienten, bei denen es sich um mit Epichlorhydrin quervernetzte Sucrose mit hohem Verzweigungsgrad handelt.

Als Separationsmedium wird besonders bevorzugt Percoll® eingesetzt, das aus Silikagelpartikeln besteht, die mit Polyvinylpyrrolidon beschichtet sind, und das nicht toxisch für Zellen ist. Es kann leicht mit gepufferten Salzlösungen auf die gewünschte Dichte verdünnt werden, ohne dass pH-Wert und Osmolalität verändert werden. Ficoll® (ein hydrophiles Polymer), ist zwar ebenfalls geeignet, muß nach Verdünnungen jedoch neu auf pH-Wert und Osmolalität eingestellt werden.

Für die Auftrennung von Knochenmark wird somit beispielsweise ein diskontinuierlicher Percoll®-Gradient mit Dichten von 1,050 bis 1,100 g/ml hergestellt. Nach Zentrifugation kann jede Fraktion mit einem charakteristischen isopycnischen Punkt einzeln entnommen und untersucht werden. Bei kontinuierlichen Gradienten dagegen ist eine genaue isopycnische Charakterisierung nicht möglich, und man erhält ein Gemisch von Zellen unterschiedlicher Dichten.

Der Gradient besteht z.B. aus 6 definierten Dichten. Jede Fraktion (F1 = geringste Dichte, F6 = höchste Dichte) wird auf Morphologie und Proliferationspotential, Expression von MSCtypischen Markern und Multipotenz in 3 Differenzierungsassays untersucht.

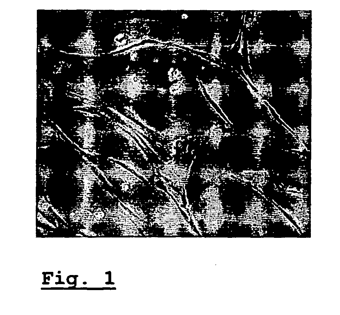

In frühen Passagen bestehen die "low-density"-Fraktionen F1 bis F3 (Dichte 1,050 bis 1,068 g/ml) überwiegend aus langgestreckten, spindelförmigen Zellen (vgl. Fig. 1, F3 in Passage 2), während in F5 und F6 bereits mit Beginn der Kultur vermehrt flache, ausgebreitete Zellen auftraten. F4 mit der Dichte 1,077 g/ml (diese Dichte wird zur Separation von MNC benutzt) enthält mit zunehmenden Passagen mehr dieser großen und flachen Zellen, während F5 und F6 mit Dichten von 1,088 und 1,100 g/ml bereits während der Primärkultur (=P0) eine erhöhte Anzahl flacher Zellen aufweist.

Die Erfindung betrifft insbesondere ein Verfahren zur Isolierung mesenchymaler Stammzellen aus Knochenmark durch Dichtegradientenzentrifugation, bei dem man zur Durchführung der Dichtegradientenzentrifugation eine isotonische Lösung von Percoll® verwendet und man die Zellen aus einer Fraktion mit einer Dichte von etwa 1,068 g/ml isoliert.

Das erfindungsgemäße Verfahren zur Isolierung mesenchymaler Stammzellen kann entweder im Rahmen einer individuellen Einzeltherapie an dem Ort bzw. der Klinik durchgeführt werden, an der der Patient behandelt wird, es ist jedoch praktikabel, die Verfahren und die anschließende Stammzell-Therapie an größeren Zentren (Good Manufacturing Practice-Zentren; GMP-Zentren) durchzuführen, da sich auf diese Weise ein gleichbleibender Qualitätsstandard garantieren lässt. Auch ist denkbar, dem Patienten nicht nur mesenchymale Stammzellen zu verabreichen, die aus seiner eigenen Knochenmark-Spende isoliert werden (autologe MSC), sondern es kommen auch allogene Zellen, d.h. von anderen Knochenmarkspendern und sonstigen freiwilligen Spendern, in Betracht, wobei darunter sowohl typisierte als auch nicht-typisierte allogene Knochenmarkspenden zu verstehen sind.

Gegenstand der Erfindung ist ferner ein Verfahren zur Herstellung eines mesenchymale Stammzellen enthaltenden pharmazeutischen Präparats, bei dem man ein vorgenanntes Verfahren zur Isolierung mesenchymaler Stammzellen aus Knochenmark durch Dichtegradientenzentrifugation durchführt und man die isolierten Stammzellen gegebenenfalls mit pharmazeutisch annehmbaren Hilfs- und Trägerstoffen formuliert.

Ferner ist auch denkbar, im Rahmen der gewerblichen Nutzung der Erfindung für die Durchführung des Verfahrens zur Isolierung der MSC erforderliche Reagenzien und Hilfsmittel bereitzustellen, beispielsweise in Form von Kits, die eine isotonische Lösung von z.B. Ficoll® oder Percoll® der Dichte 1,068 g/ml enthält. Alternativ können die Kits auch mehrere isotonische Lösungen von z.B. Ficoll® oder Percoll® unterschiedlicher Dichte enthalten. Die Lösungen unterschiedlicher Dichte liegen beispielsweise im Bereich von 1,050 g/ml bis 1,100 g/ml. Gemäß einer besonderen Ausführungsform sind die Lösungen Percoll®-Lösungen der Dichte 1,050 g/ml, 1,063 g/ml, 1,068 g/ml, 1,070 g/ml. Gemäß einer Ausführungsform der Erfindung können die Kits gegebenenfalls weitere zur Durchführung des Verfahrens erforderliche Hilfsmittel und/oder Reagenzien enthalten, wie z.B. Behältnisse, Zentrifugenröhrchen, Kulturschalen und dergleichen.

Die Erfindung wird nachfolgend anhand von Beispielen veranschaulicht:

**Beispiele**

**Materialien**

Als Ausgangslösung für die Herstellung des diskontinuierlichen Dichtegradienten wird Percoll® (Fa. Biochrom, Berlin) der Dichte 1,124 g/ml benutzt. Die Verdünnungen der Ausgangslösung mit PBS (Phosphat-gepufferte Salzlösung ohne Calcium- und Magnesiumionen, Gibco) für die gewünschten Dichten werden mit folgender Formel errechnet:

$$V[\%] = \frac{(D'-D\%) \times 10^2}{D''-D\%}$$

Wobei:

D'       gewünschte Enddichte (g/ml)
D"      hohe Ausgangsdichte (g/ml)
D%     Dichte der iso-osmolalen Verdünnungslösung (g/ml)
V%      Volumenprozent für Ausgangslösung mit hoher Dichte.

Auf diese Art wurden Separationslösungen folgender Dichten hergestellt: 1,050, 1,063, 1,068, 1,077, 1,088 und 1,100 g/ml.

**Beispiel 1**

**Durchführung der MSC-Isolierung**

Jeweils 5 ml der einzelnen Dichten wurden vorsichtig in ein 50 ml-Falcon-Röhrchen geschichtet. 10 ml Knochenmark wurden mit 10 ml PBS verdünnt und vorsichtig auf den Gradienten geschichtet.
Parallel wurde

   a) 1 ml Knochenmark mit 1 ml PBS verdünnt, auf 3 ml Ficoll® der Dichte 1,077 g/ml (als Stammzellen SC bezeichnet) in einem 15 ml-Falcon-Röhrchen als Kontrolle geschichtet und
   b) je 1 ml Knochenmark, mit 1 ml PBS verdünnt, auf 3 ml Percoll der Dichte 1,068 g/ml (bezeichnet als LD=low density) oder 1,077 g/ml (bezeichnet als MNC=mononuclear cells) in je einem separaten 15 ml-Röhrchen als Kontrolle geschichtet.

Alle Röhrchen wurden für 20 min bei Raumtemperatur mit 800g ohne Bremse zentrifugiert. Das mit PBS gemischte Plasma wurde aus den Röhrchen entfernt und jede Fraktion in ein separates Röhrchen abgenommen. Nach zweimaligem Waschen mit PBS für 10 min bei 400g werden die in F4 bis F6 enthaltenen Erythrozyten durch Hämolysepuffer entfernt, die Zellen erneut gewaschen, in Kultivierungsmedium DMEM/LG (Dulbecco's Modified Eagle Medium/niedrige Glucose, Gibco) + 1% Penicillin/ Streptomycin + 10% selektioniertes fötales Kälberserum aufgenommen und mit Trypanblau in einer Neugebauer-Kammer gezählt. Auf eine Wachstumsfläche von 25 cm$^2$ (T25, Greiner) werden 1 x 10$^7$ Zellen ausgesät. Sind in einer Fraktion weniger als 10$^7$ Zellen enthalten, werden entsprechend der Zellzahl kleinere Kulturgefäße wie 6-well Platten o.ä. benutzt. Zum Nachweis der CFU-F (colony forming unit-fibroblast; als Nachweis der Proliferationsfähigkeit einzelner Fraktionen beschrieben bei: Di-

Girolamo C et al., British J. Haematology (1999), 107: 275-281) werden 10$^6$ Zellen jeder Fraktion als auch der LD- und MNC-Kontrollzellen in je ein separates Loch einer 6-Lochplatte in 3 ml Medium eingesät. Die Zellen werden im Brutschrank bei 37°C und 5% CO$_2$ inkubiert. Nach 3 Tagen werden die nichtadhärenten Zellen entfernt, die Kulturgefäße mit PBS gewaschen und neues Medium eingefüllt.
Die Zellen werden zweimal wöchentlich durch Mediumwechsel gefüttert und bis zu einer Konfluenz von 80-90% (visuelle Bewertung im Mikroskop) inkubiert. Zu diesem Zeitpunkt wird die Kultur mit P0 als Primärkultur bezeichnet. Für die Passagierung wird das gesamte Medium entnommen, die Wachstumsfläche mit PBS gewaschen, für 5 min mit 0,25% Trypsin-EDTA inkubiert und anschließend unter Zugabe von Medium resuspendiert und gezählt. 500 Zellen/cm$^2$ werden in neue T25 oder T75 zur Weiterführung der Kulturen eingesät und jetzt als P1 bezeichnet. Die CFU-F werden nach 14 Tagen Inkubation mit PBS gewaschen und mit 1% Kristallviolett gefärbt.

**Beispiel 2**

**Differenzierungsexperimente**

Für Differenzierungsexperimente werden 6 x 10$^3$ Zellen pro Loch einer 24-well Platte ausgesät, je 4 Löcher für die Induktion der osteogenen und adipogenen Differenzierung.
Nach Erreichen der Konfluenz wird die **adipogene** Differenzierung, wie bei Pittenger et al. 1999 (loc. cit.) beschrieben, induziert. Dazu wird das Medium mit 1 μM Dexamethason + 0,5 mM Isobutylmethylxanthin + 100 μM Indomethacin + 10 μM Insulin versetzt und die Zellen für 3-4 Tage inkubiert. Für einen Tag werden die Zellen mit Medium nur mit Insulin zur Erhaltung inkubiert. Kontrolllöcher werden jeweils ohne die Zusätze kultiviert, um eventuelle Spontandifferenzierungen nachzuweisen. Dieser Zyklus von Induktion und Erhaltung wird sechsmal wiederholt. Anschließend werden die Zellen mit PBS gewaschen, für 10 min mit 4% Formalin fixiert, kurz mit 50% Ethanol gewaschen und für 15-30 min mit Sudan-Rot B gefärbt. Nach kurzem Waschen mit 50% Ethanol wird gegengefärbt mit Hämalaun für 5 min, gewässert für 1 min mit Leitungswasser und anschließend mit flüssigem Paraffinöl haltbar gemacht.
Für die **osteogene** Differenzierung werden konfluente Kulturen, wie bei Jaiswal et al. 1997 (loc. cit.) beschrieben, induziert. Dazu werden die Kulturen mit 10$^{-7}$ M Dexamethason + 50 μM Ascorbinsäure + 10 mM β-Glycerolphosphat versetztem Medium inkubiert und dieses Medium nach 3-4 Tagen ausgetauscht. Die Kontrollkulturen erhalten Medium ohne Induktionskomponenten. Nach 2-3 Wochen werden die Mineral-Ablagerungen aus Calcium nach der von-Kossa-Methode gefärbt (von Kossa, J. et al. (1901) Beit. Path. Anat. 29: 163). Dazu werden die Zellen mit PBS gewaschen, für 10 min mit 4% Formaldehyd fixiert, 1 x mit PBS und 2 x mit aqua dest. gewaschen und lufttrocknen gelassen. Anschließend wird für 10 min mit Silbernitrat unter UV-Licht gefärbt, 2-3 x mit aqua dest. gewaschen, mit Hämalaun für 1 min gegengefärbt und nach Wässern mit Leitungswasser mit flüssigem Paraffinöl eingedeckt.

Die **chondrogene** Differenzierung erfolgt modifiziert nach der Methode von Shakibaei et al. 1997 (loc. cit.). Dazu werden 3 x 10$^4$ Zellen in einem Eppendorfröhrchen in 20 µl 2%-igem Alginat aufgenommen. Die Alginat-Zellsuspension wird in 0,1 M CaCl$_2$ in 6-well Platten als Tropfen gegeben und geliert dort für 10 min bei Raumtemperatur. Nach 3-maligem Waschen mit 0,15 M NaCl wird 2 x mit Medium gewaschen und die Alginatkugeln in Medium für 7 Tage im Brutschrank bei 37°C und 5% CO$_2$ inkubiert mit ein- bis zweimaligem Mediumwechsel. Die Alginat-Kugeln werden *in toto* für 1 h in 10% Formalin bei Raumtemperatur fixiert, für 5 min in 2% Essigsäure gewaschen und für 24 h bei RT in Alcian-Blau-Lösung gefärbt. Dabei werden spezifisch die Proteoglykane, die durch die Zellen als Matrix gebildet wurden, angefärbt. Nach 3-maligem Waschen in a.d. werden die Alginat-Kugeln mit aufsteigender Alkoholreihe zu 90% Ethanol jeweils 10 min dehydriert, für 5-10 min in Xylol dehydriert und mit Entellan unter leichtem Druck eingebettet.

**Beispiel 3**

**Phänotypische Untersuchung auf Oberflächenmarker**

Die Zellen jeder Passage werden weiterhin einer phänotypischen Untersuchung auf Oberflächenmarker unterzogen. Dazu wurden folgende Antikörper verwendet: CD34-PE (Phycoerythrin), CD45-PE, CD90-FITC (Fluoroisothiocyanat), CD105-FITC, CD59-FITC und die entsprechenden Isotypkontrollen Maus-IgG1-PE, Maus-IgGl-FITC und Maus-IgG2a-FITC.

Mindestens 5-10 x 10$^4$ Zellen wurden mit der vom Hersteller angegebenen Menge Antikörper in 50 µl FACS-Puffer (PBS + 2% FCS + 0,1% Natrium-Azid; FCS = fötales Kälberserum) für 20 min bei Raumtemperatur inkubiert und anschließend mit FACS-Puffer gewaschen. Die gefärbten Zellen werden in 3-400 µl FACS-Puffer resuspendiert und an einem FACScan/Becton Dickinson der Analyse unterzogen. Dabei werden die Einstellungen für Vorwärts- und Seitwärts-Streueigenschaften als auch der Fluoreszenz mit der Isotypkontrolle vorgenommen. Die Auswertung erfolgt mit der CellQuest-Software von Becton Dickinson.

Die erfindungsgemäßen Zellen zeigen selbst in schlecht wachsenden Kulturen bzw. schlecht wachsenden Fraktionen mindestens 20 Verdopplungen und sind auf das sehr sorgfältig selektierte FCS zurückzuführen. In den Ansätzen wurden 500 Zellen/cm$^2$ ausplattiert. Bis zur Konfluenz von 80-90% verdoppeln sich die Zellen in Abhängigkeit von der Passage ca. 2-6 mal. Bei der Selektionierung des FCS wurde auf Wachstum, Phänotyp und Differenzierung in drei Richtungen untersucht. Wachstumskurven wurden bis Passage 4 erstellt. In Passage 4 wurden der Phänotyp und die Differenzierung in drei Richtungen (osteogene, chondrogene, adipogene Richtung) untersucht. Waren Phänotyp und Differenzierungspotential bei verschiedenen Seren gleich, wurde dem schnelleren Wachstum der Vorrang gegeben.

**Ergebnisse**

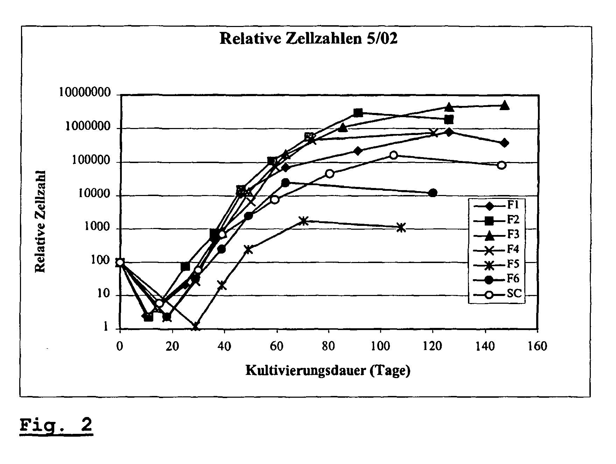

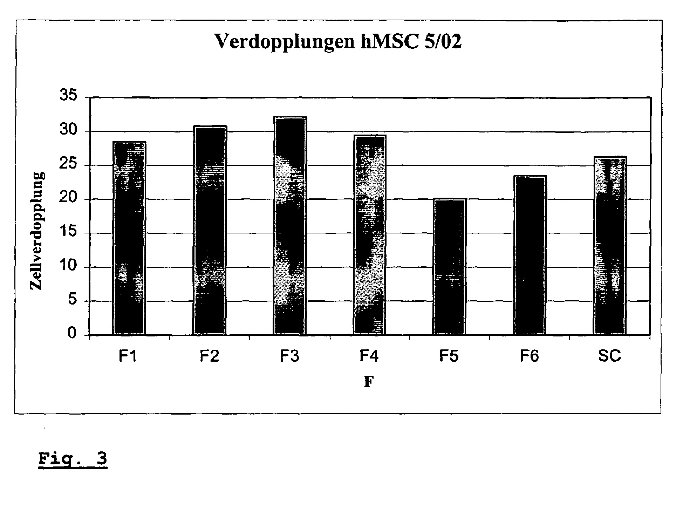

Der Vergleich der **Proliferationsraten** der einzelnen Fraktionen zeigte, dass Zellen, die mit Dichten von 1,05 bis 1,068 g/ml (entspricht F1 bis F3) separiert wurden, mehr Zellteilungen (Fig. 2) und damit mehr Verdopplungen (Fig. 3) bis zum Stillstand der Kultur erbringen als Zellen höherer Dichten. SC bezeichnet die mitgeführte Kontrolle von Zellen über Ficoll® aufgereinigt. Die in Fig. 3 gezeigten Verdopplungen sind aus den Zellzahlen der Probe aus Fig. 2 berechnet. In Abhängigkeit von der Qualität des Spenderknochenmarks sind die Proliferationseigenschaften der definierten Fraktionen nicht immer identisch, zeigen jedoch immer die Prominenz der "low-density"-Fraktionen. Der Unterschied in den Verdopplungsraten zwischen F3 als "low-density"-Zellen und F4 als MNC kann bis zu 5 Verdopplungen betragen, d.h. aus z.B. 10$^{13}$ Zellen würden 3,2 x 10$^{14}$ Zellen werden.

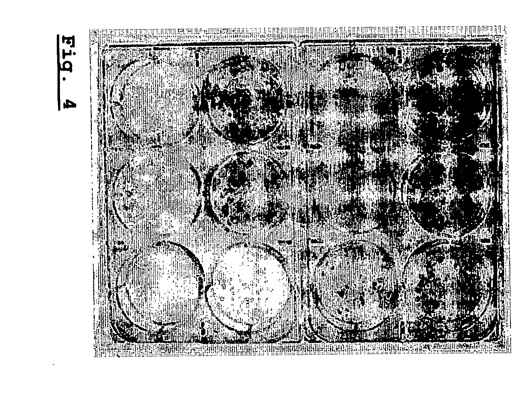

Diese Ergebnisse werden durch die Untersuchung von CFU-F der einzelnen Fraktionen und im Vergleich von LD- und MNC-Zellen bestätigt (Fig. 4). Deutlich zu sehen sind eine Vielzahl von CFU-F in den Fraktionen F1 bis F3 und stark reduzierte Zahlen in F4. In F5 und F6 bilden sich kaum noch CFU-F. In den LD-Zellen dagegen ist die höchste Anzahl dieser Kolonie-bildenden Zellen zu finden und deutlich mehr als in den einzelnen Fraktionen F1, F2 und F3 als auch den MNC-Zellen.

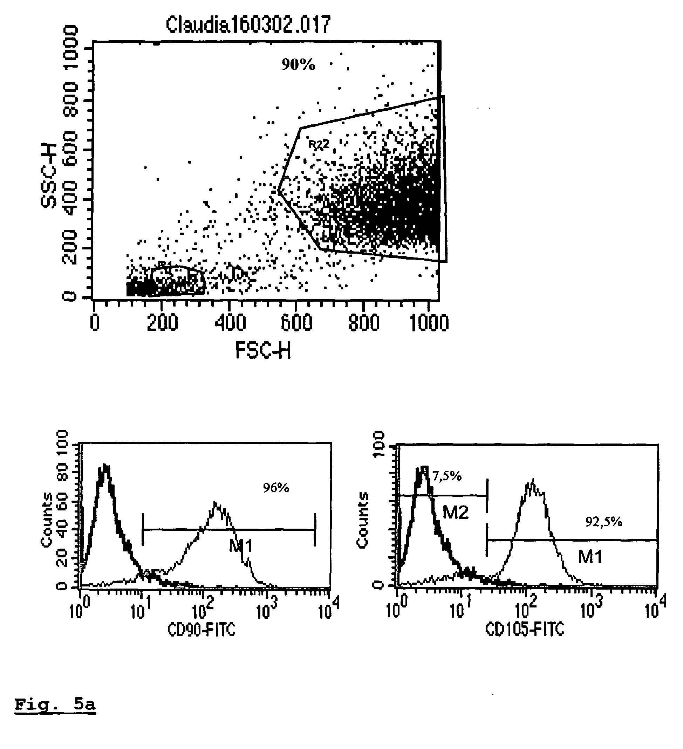

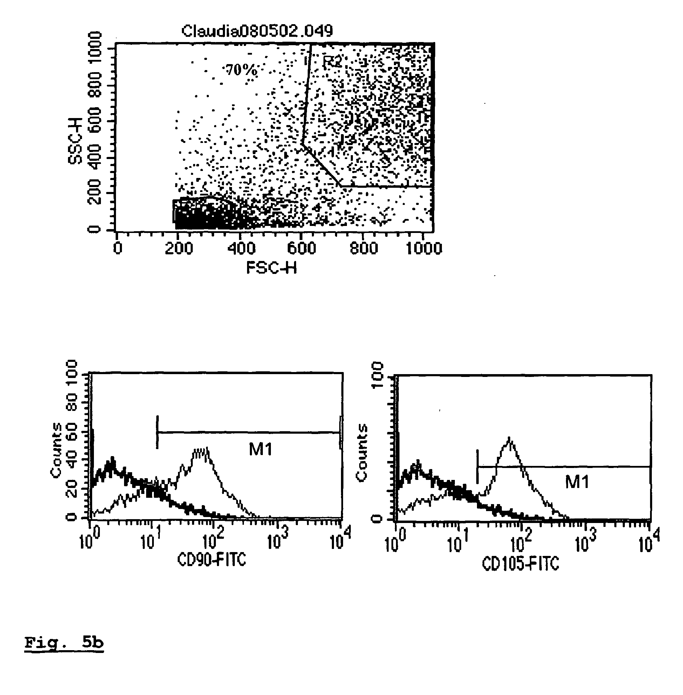

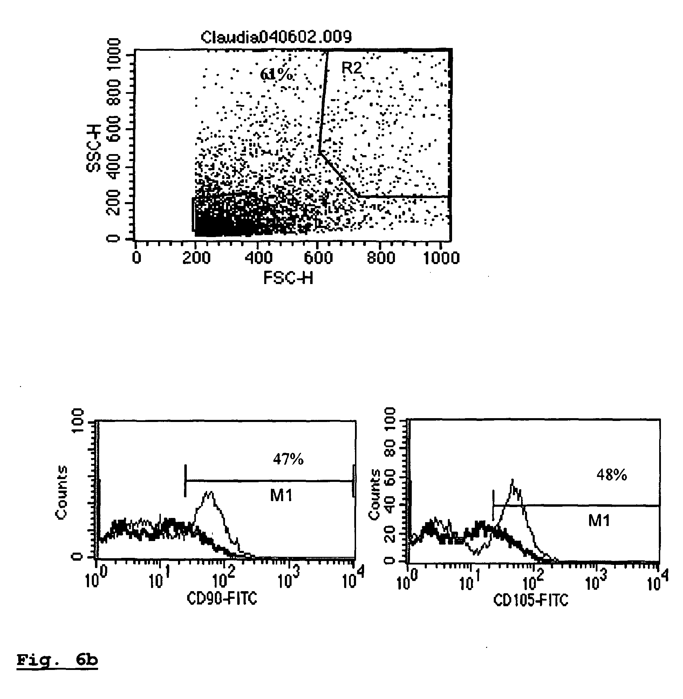

Die Untersuchungen zum **Phänotyp** der in Fraktionen aufgetrennten MSC zeige Populationen, die negativ für die hämatopoetischen Marker CD45 und CD34 sind (nicht dargestellt), jedoch positiv für CD90 (Thy-1, Marker für frühe Progenitorzellen), CD105 (Endoglin, spezifischer Marker für MSC) und CD59 (Sca-1 = "Stem cell antigen"-Homolog, Marker für frühere Stammzellen, nicht dargestellt). In der FACS-Analyse können die MSC in zwei Populationen unterteilt werden: eine kleine Population R1 mit ca. 2-5% besteht aus kleinen, gering granulierten Zellen und eine prominente Population R2, die aus großen, stark granulierten Zellen besteht (Fig. 5a, 6a, jeweils linkes Histogramm). Die R1-Zellen sowohl der Fraktionen der geringen Dichte (Fig. 5a, b) als auch der mit höherer Dichte (Fig. 6a, b) sind negativ für CD90 und CD105 (kleiner Peak der schwarzen Kurve; grau: Isotypkontrolle), während die Hauptpopulation R2 positiv ist für beide Marker.

Mit diesem Antigen-Profil erscheinen die R1-Zellen eher eine stark unreife Population darzustellen. Die Zahl der Zellen in der R2-Population nimmt mit steigender Kultivierungszeit ab. In den Histogrammen erscheinen mehr Zellen in R1, was jedoch auf eine Zunahme von apoptotischen (sterbenden) Zellen und Debris zurückzuführen ist. Mit fortschreitender Kultivierung nimmt die Zahl der für beide gezeigten Marker positiven Zellen deutlich ab. Die Abnahme der CD90- und CD105-positiven Zellen ist in Fraktionen höherer Dichte (Fig. 6b versus Fig. 6a) prominenter als in Fraktionen niedrigerer Dichten (Fig. 5b versus Fig. 5a). Korreliert man die Reduktion der Expression MSCtypischer Marker mit der Fähigkeit der Zellen, in die osteogene Richtung zu differenzieren, so scheint diese Differenzierung nicht von der Expression der Marker auf allen Zellen abzuhängen.

## Beschreibung der Figuren

Fig. 1:     Spindelförmige MSC in Fraktion 3 in der 2. Passage.

Fig. 2:     Relative Zellzahlen anhand eines Beispiels aus 3 Versuchen.

Fig. 3:     Verdopplungen einzelner Fraktionen eines Beispiels aus 3 Versuchen.

Fig. 4:     CFU-F der einzelnen Fraktionen F1 bis F6 im Vergleich mit LD- und MNC-Zellen eines Beispiels aus 5 Versuchen.

Fig. 5a:    Streueigenschaften und Expression von Oberflächenmarkern an einem Beispiel von MSC der Fraktion F3 in der 2. Passage.

Fig. 5b:    Streueigenschaften und Expression von Oberflächenmarkern an einem Beispiel von MSC der Fraktion F3 in der 7. Passage.

Fig. 6a:    Streueigenschaften und Expression von Oberflächenmarkern an einem Beispiel von MSC der Fraktion F6 in der 2. Passage.

Fig. 6b:    Streueigenschaften und Expression von Oberflächenmarkern an einem Beispiel von MSC der Fraktion F6 in der 7. Passage.

## Patentansprüche

1.  Verfahren zur Herstellung einer Präparation humaner mesenchymaler Stammzellen aus Knochenmark durch Dichtegradientenzentrifugation, **dadurch gekennzeichnet, daß** man die Zellen als Präparation, die mindestens 70% dieser Zellen enthält, aus einer Fraktion mit einer Dichte von 1,050 - 1,070 g/ml isoliert.

2.  Verfahren nach Anspruch 1, **dadurch gekennzeichnet, daß** man zur Durchführung der Dichtegradientenzentrifugation eine isotonische Lösung von Ficoll® oder Percoll® verwendet.

3.  Verfahren nach Anspruch 2, **dadurch gekennzeichnet, daß** man Percoll® verwendet und die Dichte 1,068 g/ml beträgt.

4.  Mesenchymale Stammzell-Präparation, erhalten nach einem Verfahren der Ansprüche 1 bis 3.

5.  Pharmazeutisches Präparat, enthaltend eine Präparation nach Anspruch 4.

6.  Verfahren zur Herstellung eines mesenchymale Stammzellen enthaltenden pharmazeutischen Präparats, **dadurch gekennzeichnet, daß** man ein Verfahren nach den Ansprüchen 1 bis 3 durchführt und man die isolierten Stammzellen gegebenenfalls mit pharmazeutisch annehmbaren Hilfs- und Trägerstoffen formuliert.

7.  Verwendung einer Lösung von Ficoll® oder Percoll® der Dichte 1,068 g/ml zur Durchführung des Verfahrens nach den Ansprüchen 1 bis 3.

## Claims

1.  Method for the production of a preparation of human mesenchymal stem cells from bone marrow by density gradient centrifugation, wherein the cells are isolated as a preparation containing at least 70% of these cells from a fraction with a density of 1.050 - 1.070 g/ml.

2.  Method according to claim 1, wherein an isotonic solution of Ficoll® or Percoll® is used to perform the density gradient centrifugation.

3.  Method according to claim 2, wherein Percoll® is used and the density is 1.068 g/ml.

4.  Mesenchymal stem cell preparation, obtained according to a method of claims 1 to 3.

5.  Pharmaceutical preparation, containing a preparation according to claim 4.

6.  Method for the production of a pharmaceutical preparation containing mesenchymal stem cells, wherein a method according to claims 1 to 3 is performed and, if necessary, the isolated cells are formulated with pharmaceutically acceptable excipients and carriers.

7.  Use of a solution of Ficoll® or Percoll® of the density 1.068 g/ml to perform the method according to claims 1 to 3.

**Revendications**

1. Procédé de fabrication d'une préparation de cellules souches mésenchymateuses à partir de moëlle osseuse par centrifugation par gradient de densité, **caractérisé en ce qu'**on isole une préparation de cellules qui contient au moins 70% de ces cellules à partir d'une fraction ayant une densité de 1,050-1,070 g/ml.

2. Procédé selon la revendication 1, **caractérisé en ce qu'**on utilise une solution isotonique de Ficoll® ou de Percoll® lors de la mise en oeuvre de la centrifugation par gradient de densité.

3. Procédé selon la revendication 2, **caractérisé en ce qu'**on utilise du Percoll® et **en ce que** la densité est de 1,068 g/ml.

4. Préparation de cellules souches mésenchymateuses, obtenue par le procédé de l'une des revendications 1 à 3.

5. Formulation pharmaceutique, comprenant une préparation selon la revendication 4.

6. Procédé d'obtention d'une préparation pharmaceutique contenant des cellules souches mésenchymateuses, **caractérisé en ce qu'**on conduit un procédé selon l'une des revendications 1 à 3 et **en ce qu'**on formule les cellules souches isolées le cas échéant avec des adjuvants ou véhicules pharmaceutiquement acceptables.

7. Utilisation d'une solution de Ficoll® ou de Percoll® ayant une densité de 1,068 g/ml pour la mise en oeuvre du procédé selon les revendications 1 à 3.

Fig. 1

**Fig. 2**

**Verdopplungen hMSC 5/02**

Fig. 3

EP 1 651 757 B1

Fig. 4

12

**Fig. 5a**

Fig. 5b

14

**Fig. 6a**

**Fig. 6b**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **AZIZI et al.** *Proc Natl Acad Sci USA,* 1998, vol. 95, 3908-13
- **PHINNEY et al.** *J Cell Biochem,* 1999, vol. 75, 424-36
- **DIGIROLAMO et al.** *Br J Haematol,* 1999, vol. 107, 275-81
- **MURAGLIA et al.** *J Cell Science,* 2000, vol. 113, 1161-66
- **COLTER et al.** *Proc Natl Acad Sci USA,* 2000, vol. 97, 3213-18
- *Proc Natl Acad Sci USA,* 2001, vol. 98, 7841-45
- **QUIRICI et al.** *Exp Hematol,* 2002, vol. 30, 783-91
- **MAJUMDAR et al.** *J Cell Physiol,* 1998, vol. 176, 57-66
- **MACKAY et al.** *Tissue Engineering,* 1998, vol. 4, 415-28
- **PITTENGER et al.** *Science,* 1999, vol. 284, 143-7
- **MOSCA et al.** *Clin Orthop Rel Res,* 2000, vol. 379S, 71-90
- **KOC et al.** *Bone Marrow Transpl,* 2002, vol. 30, 215-22
- **TOMA et al.** *Circulation,* 2002, vol. 105, 93-8
- **LENNON et al.** *In Vitro Cell Dev Biol,* 1996, vol. 32, 602-11
- **BRUDER et al.** *J Cell Biochem,* 1997, vol. 64, 278-94
- **JAISWAL et al.** *J Cell Biochem,* 1997, vol. 64, 295-312
- **FLEMING et al.** *Developm Dynamics,* 1998, vol. 212, 119-32
- **LIECHTY et al.** *Nat Med,* 2000, vol. 6, 1282-6
- **SHAKIBAEI et al.** *Cell Biol Internat,* 1997, vol. 21, 75-86
- **DIGIROLAMO C et al.** *British J. Haematology,* 1999, vol. 107, 275-281